# EUROPÄISCHE PATENTSCHRIFT

(11) **EP 0 585 695 B1**
(45) Veröffentlichungstag und Bekanntmachung des Hinweises auf die Patenterteilung: **17.06.1998**
(21) Anmeldenummer: 93112998.5
(22) Anmeldetag: 13.08.1993
(51) Int. Cl.: C12M 3/06, C12M 3/04, C12M 1/12, C12M 1/10

(54) **Kulturgefäss für Zellkulturen**
Device for cell culture
Dispositif de culture des cellules

(30) Priorität: 02.09.1992 DE 4229325
(43) Veröffentlichungstag der Anmeldung: 09.03.1994
(73) Patentinhaber: Heraeus Instruments GmbH & Co. KG, 63450 Hanau (DE)
(72) Erfinder: Falkenberg, Frank W., Prof.Dr., D-85456 Witten (DE); Nagels, Hans Otto, Dr., D-37120 Bovenden (DE); Köhn, Heinz-Gerhard, Dr., D-37127 Dransfeld (DE)
(74) Vertreter: Kühn, Hans-Christian

(56) Entgegenhaltungen:
- EP-A- 0 180 165
- WO-A-91/02555
- US-A- 4 717 668

## Beschreibung

Die Erfindung betrifft ein Kulturgefäß für Zellkulturen mit mindestens einer die Zellkultur aufnehmenden Zellkultur-Kammer, die durch eine Dialyse-Membran von einem einzubringenden Nährmedium abgetrennt ist, wobei durch die Dialyse-Membran Nährstoffe in die Zellkultur-Kammer transportiert und von der Zellkultur-Kammer Stoffwechselprodukte in das Nährmedium abtransportiert werden und mit einer Zuund Abführung für die bei der Zellkultivierung benötigten und entstehenden Gase.

Derartige Kulturgefäße können beispielsweise für die in vitro-Gewinnung monoklonaler Antikörper eingesetzt werden. Monoklonale Antikörper werden heutzutage für zahlreiche Anwendungszwecke in Diagnostik, Therapie und biologisch-medizinischer Forschung üblicherweise nach Methoden der Hybridomtechnologie produziert. Als Hybridomzellen werden immortalisierte Hybride aus Antikörper-produzierenden Zellen und Myelomzellen bezeichnet. Die von den Hybridomzellen produzierten Antikörper, die sich durch eine hohe Spezifität auszeichnen, werden als "monoklonale" Antikörper bezeichnet.

Zur in vitro-Gewinnnung der Antikörper werden diese Hybridomzellen in bestimmten flüssigen Medien kultiviert, deren Zusammensetzung möglichst genau der des Blutes entspricht. Unter anderem enthalten diese Medien Salze, Zucker, Vitamine, Aminosäuren und ein auf Natrium-Hydrogencarbonat (NaHCO₃) basierendes Puffersystem. Üblicherweise werden die Hypridomzellen in einer Brutschrankatmosphäre mit hoher Luftfeuchtigkeit und einem CO₂-Gehalt, der mit dem im Medium enthaltenen NaHCO₃ im Gleichgewicht steht, kultiviert.

Für viele Anwendungszwecke der biologisch-medizinischen Grundlagenforschung, wie auch in der klinischen Diagnostik, sind die so nach konventionellen stationären in vitro-Methoden in Form von Gewebekulturüberstand gewonnenen monoklonalen Antikörper sehr gut geeignet. Für eine Reihe von Anwendungen, für die man monoklonale Antikörper in sehr reiner und hochkonzentrierter Form benötigt, sind die so gewonnenen monoklonalen Antikörper erst nach aufwendiger Weiterbearbeitung geeignet. Bei der in vivo-Produktionsform (Aszites-Flüssigkeit) sind im Ausgangsprodukt die monoklonalen Antikörper bereits in sehr hohen Konzentrationen (bis zu 20 mg/ml) enthalten. Bei der Gewinnung von monoklonalen Antikörpern durch die üblichen stationären in vitro-Produktionsformen werden jedoch nur Konzentrationen von ca. 0,01 bis 0,10 mg/ml erzielt.

Um die Herstellung monoklonaler Antikörper in höherer Konzentration und höherer Reinheit auch in vitro zu ermöglichen, wurde eine Vorrichtung in Form eines rollflaschenähnlichen Kulturgefäßes und ein Verfahren vorgeschlagen, bei dem eine "Versorgungs-Kammer" mit Nährstoffen für die zu versorgenden Zellen und mehrere darin angeordnete "Produktions-Kammern", in denen das Zellwachstum stattfindet und in denen die monoklonalen Antikörper produziert werden, voneinander durch semipermeable Dialyse-Membranen getrennt sind. Durch die semipermeable Dialyse-Membran hindurch werden die Zellen von der "Versorgungs-Kammer" aus mit Nährstoffen versorgt, während Abbau- und Stoffwechselprodukte ebenfalls durch die Dialyse-Membran aus den "Produktions-Kammern" in die "Versorgungs-Kammer" abgeführt werden. Diese Vorrichtung ist unter dem Namen "Bochumer Glasmaus" bekannt geworden. Dieses Kulturgefäß für Zellkulturen ist beispielsweise in dem Manuskript zu dem Poster-Vortrag "The Glassmmouse; A Rollerbottle-like Apparatus for Culturing Hybridomas in Dialysis Bags" von T. Hengelage, F. Haardt und F. W. Falkenberg, ausgestellt auf der Fachtagung "1991 World Congress on Cell and Tissue Culture", Anaheim, Kalifornien, vom 16. bis 20. Juni 1991, beschrieben. Das bekannte Kulturgefäß für Zellkulturen besteht aus einem Glasrohr mit einem Außendurchmesser von 120 mm, von dem die Enden in Form von Flanschen nach außen umgebogen sind. Die Länge des Glasrohres inklusive der Flansche beträgt 320 mm. Die Stirnseiten des Glasrohres sind mit 15 mm dicken PMMA-Scheiben verschlossen. Eine der PMMA-Scheiben weist 5 Durchgangsbohrungen auf, wovon eine in der Längsachse des Gefäßes ausgebildet und mit einem Stopfen verschlossen ist, der wiederum 2 kleinere Öffnungen aufweist, die als Einlaß für ein CO₂-Luft-Gemisch in das Gefäß und zum Druckausgleich dienen. Hierzu ist durch eine der beiden Öffnungen ein Edelstahl rohr mit einem Innendurchmesser von 1 mm hindurchgeführt, das bis zur gegenüberliegenden Stirnseite des Glasrohres reicht und durch das dem Inneren des Gefäßes über ein Sterilfilter das CO₂-Luft-Gemisch zugeführt wird. Die restlichen 4 Bohrungen der PMMA-Scheibe, die die zentrale Bohrung umgeben, dienen zur Einführung von Dialyse-Schläuchen, die in das Kulturgefäß hineinragen und deren Wandungen jeweils von einer semipermeablen Dialyse-Membran gebildet wird. In diese Dialyse-Schläuche werden die zu kultivierenden Zellkultur-Ansätze gefüllt; sie dienen als Produktions-Kammern, während der Innenraum des Kulturgefäßes im übrigen als Versorgungs-Kammer für die Zellen dient und bis etwa 40% des Volumens mit Nährmedium gefüllt ist. Durch die semipermeable Dialyse-Membran hindurch werden die Zellen von der Versorgungskammer aus mit Nährstoffen versorgt, während Abbau- und Stoffwechselprodukte ebenfalls durch die Dialyse-Membran abgeführt werden. Um ein Rotieren des Kulturgefäßes um seine Längsachse zu ermöglichen, kann dieses mit einer dichten Drehdurchführung versehen sein, durch die die Zuleitung für das CO₂-Luft-Gemisch hindurchgeführt ist.

Diese Vorrichtung, bei der die in die Produktions-Kammer eingeschlossenen Zellen von der semipermeablen Dialyse-Membran umgeben sind, ermöglicht die Kultivierung von Hypridomzellen über längere Zeiträume und in hoher Dichte (mehr als 10⁷ Zellen/ml). Bei dem bekannten Kulturgefäß handelt es sich jedoch um eine relativ aufwendige und kompliziert zu handhabende Vorrichtung, deren Bau einen gewissen Aufwand erfordert, den nicht jede Laborwerkstatt erbringen kann. Bei dem bekannten Kulturgefäß erfolgt die Versorgung mit dem für den Stoffwechsel der Zellkultur und für die Einstellungen der physiologischen Bedingungen notwendigen Gasen durch Einleiten des die umgebende Atmosphäre bildenden Gasgemisches in die Versorgungs-Kammer, wobei sich der Sauerstoff im Nährstoffmedium physikalisch löst und von dort durch die Dialysemembran in die Produktions-Kammer transportiert wird. Der Transport des Sauerstoffs von der Versorgungs-Kammer durch die Dialyse-Membran in die Zellkultur-Kammer ist zwar nicht sehr effektiv, reicht jedoch bis zu Zelldichten von ca. 10⁷ Zellen/ml aus. Für höhere Zelldichten bedarf es einer Verbesserung der Sauerstoffversorgung. Da bei hoher Zelldichte der Sauerstoffbedarf der Zellen so groß ist, daß der Gehalt an Sauerstoff in der Zellkultur-Kammer in wenigen Minuten, der in der Versorgungs-Kammer in weniger als 1 Stunde verbraucht ist, ist es notwendig, Sauerstoff aus der Gasphase durch Eintrag in das Nährmedium der Versorgungs-Kammer nachzuliefern. Als Schwachpunkt des bekannten Kulturgefäßes hat sich auch erwiesen, daß es durch die kontinuierliche Begasung mit dem CO₂-Luft-Gemisch über die Drehdurchführung zu Infektionen der Zellkulturen kommen kann.

Aus der US 4,717,668 ist weiterhin ein rollflaschenähnliches Kulturgefäß aus Kunststoff bekannt, das flexible O₂- und CO₂-durchlässige Außenwände aufweist, die durch Ringelemente verstärkt sind. Zum Befüllen und Entleeren des annähernd zylindrischen Kulturgefäßes sind an einer Stirnseite steril verschließbare Einfüll- und Absaugöffnungen vorgesehen. Das Kulturgefäß besteht nur aus einer Kammer, in der die Kultivierung einer Zellen mit Hilfe einer Nährlösung stattfindet. Derartige Ein-Kammer-Kulturgefäße sind für die Zellkultivierung mit einer Ausbeute von 10⁷ Zellen/ml und mehr bei weitem nicht ausreichend. Die Zuführung von Nährstoffen und die Abführung von nicht-gasförmigen Stoffwechselprodukten aus der Zellkultur ist in dem Zellkulturgefäß nach US 4,717,668 nicht möglich. Über die gasdurchlässige Außenwand des Ein-Kammer-Kulturgefäßes kann nur O₂ zugeführt werden bzw. der CO₂ - Austausch stattfinden, was die Zellausbeute bei dem aus US 4,717,668 bekannten Kulturgefäß stark einschränkt. Weiterhin wird die Zellausbeute dadurch begrenzt, daß es im Innern der Produktionskammer keine Mischelemente gibt, die beim Rollieren die Durchmischung intensivieren könnten.

Der vorliegenden Erfindung liegt die Aufgabe zugrunde ein Kulturgefäß für die Erzeugung von Zellkulturen mit hoher Zelldichte bereitzustellen, bei dem das Zellwachstum nicht durch eine Unterversorgung der Zellen mit Sauerstoff limitiert wird, das preisgünstig herstellbar und einfach handhabbar ist und bei dem die Gefahr von Infektionen vermindert ist.

Diese Aufgabe wird ausgehend von dem eingangs charakterisierten Kulturgefäß erfindungsgemäß dadurch gelöst, daß als Gas-Zu- und Abführung eine für Flüssigkeiten und die Zellkulturen kontaminierende Keime undurchlässige Gasaustausch-Membran vorgesehen ist, die die Zellkultur-Kammer teilweise begrenzt. Dadurch, daß als Gas-Zu- und Abführung eine gasdurchlässige, aber für die die Zellkultur kontaminierenden Keime und Flüssigkeiten undurchlässige Membran vorgesehen ist, sind Infektionen der Zellkultur bzw. der Zellkulturen, die über die Gas-Zu- und Abführung eingeschleppt werden, nahezu ausgeschlossen. Die Gas-Zu- und Abführung über eine Gasaustausch-Membran kommt ohne Gas-Zu- leitungen bzw. -Ableitungen in Form von Rohrleitungen oder -schläuchen aus. Das Kulturgefäß ist daher sehr einfach handhabbar und beispielweise einfach als Rollflasche einsetzbar. Da der Zellkultur-Kammer die für die Gasversorgung notwendigen Gase direkt zugeführt, bzw. die gasförmigen Stoffwechselprodukte durch die Gasaustausch-Membran von der Zellkultur-Kammer direkt abgeführt werden, wird ein schneller und durch entsprechende Einstellung der das Kulturgefäß umgebenden Atmosphäre und des Drucks außerhalb der Zellkultur-Kammer unmittelbar beeinflußbarer Gasaustausch erreicht. Als die für die Zellatmung notwendigen Gase gelten dabei in erster Linie Sauerstoff und das beim Verbrauch des Sauerstoffs durch die Zellkultur entstehende Kohlendioxid. Durch die Einstellung dieser Gase in der Atmosphäre, die die Gasaustausch-Membran umgibt, kann deren Konzentration in der Zellkultur-Kammer unmittelbar vorgegeben werden. Die Gesamtfläche, das Material und die Dicke der Gasaustausch-Membran sind so zu wählen, daß eine Sauerstoffversorgung gewährleistet wird, die den Sauerstoffbedarf bei den angestrebten hohen Zelldichten deckt. Geeignete Gasaustausch-Membran-Geometrien und Gasdurchlässigkeiten lassen sich anhand weniger Versuche ermitteln.

Als Zellkulturen kommen beispielsweise Hybridomzellen, Tumorzellen oder transfizierte Tumorzellen in Frage.

Die Gasaustausch-Membran erfüllt in erster Linie die Aufgabe, den für die Zellkultivierung erforderlichen Gasaustausch zu gewährleisten. Sie kann daher, im Gegensatz zu der Dialyse-Membran bei dem bekannten Kulturgefäß, durch die sowohl die Gasversorgung, als auch die Versorgung mit nicht gasförmigen Nährstoffen sicherzustellen ist, für die Erfüllung dieser Aufgabe optimiert werden. Außerdem kann die Gasaustausch-Membran auf der der Zellkultur abgewandten Seite dem Gas direkt, das heißt ohne eventuell störende Zwischenschichten oder Oberflächenfilme, ausgesetzt werden. Druckänderungen oder Änderungen der Gas-Zusamensetzung werden an die Gasaustausch-Membran direkt weitergegeben.

Für die Ausbildung der Gasaustausch-Membran haben sich Materialien als geeignet erwiesen, die für Sauerstoff einen Durchlässigkeitskoeffizienten von mindestens 1 x 10¹⁹ m²/s x Pa, vorteilhafterweise mindestens 5 x 10¹⁹ m²/s x Pa, aufweisen. Als besonders gut geeignete Materialien zur Ausbildung der Gasaustausch-Membran haben sich Silikon und mikroporöses, hydrophobes oder hydrophobiertes Material erwiesen. Um eine ausreichende Sauerstoffversorgung zu gewährleisten, werden möglichst dünne Gasaustausch-Membranen bevorzugt, bewährt haben sich Membranen mit einer Dicke zwischen 0,1 mm und 1 mm. Eine Silikon-Membran ist besonders preisgünstig und durch Spritzgußverfahren in jeder beliebigen Form herstellbar. Im Handel sind Silikone in vielen Dicken, Formen und definierten Gasdurchlässigkeiten erhältlich. Sie weist eine hohe Zerreißfestigkeit und eine gute chemische Beständigkeit gegenüber den üblicherweise bei der Zellkultivierung verwendeten Medien auf und ist insofern auch besonders einfach handhabbar. Besonders vorteilhaft ist weiterhin die einfache Sterilisierbarkeit einer Membran aus Silikon; insbesondere ist sie besonders wirksam und ohne wesentliche Formänderung im Autoklaven sterilisierbar. Sie ist daher auch mehrfach verwendbar. Als Material für die Ausbildung der Gasaustausch-Membran hat sich auch mikroporöses, hydrophobes Polytetrafluorethylen (PTFE) als günstig erwiesen. Sein hydrophober Charakter gewährleistet die Undurchlässigkeit der Gasaustausch-Membran für wässrige Medien. Bei gegebener Gaspermeabilität hängt die erforderliche Geometrie der Gasaustausch-Membran von dem durch die Zellatmung verursachten Gasbedarf sowie von den Partialdrücken der an der Zellatmung beteiligten Gase ab, insbesondere von dem von außen auf sie wirkenden Sauerstoffpartialdruck. Bei einem Außendruck von 1 atm und einer Brutschrankatmosphäre mit einem Sauerstoffpartialdruck etwa entsprechend der Luft, haben sich für Zellkulturen von 35 ml und 10⁷ Zellen pro Milliliter Zellkulturansatz, Gasaustausch-Membranen mit einer Fläche von mindestens 5 cm² als geeignet erwiesen.

Besonders hohe Zelldichten sind mit Kulturgefäßen erreichbar, bei denen in der Zellkultur-Kammer Mischelemente eingesetzt sind. Die Mischelemente bewirken eine gute Durchmischung der Zellkultur und dadurch eine gleichmäßige Versorgung der gesamten Zellkultur mit Nährstoffen. Als besonders einfach handhabbar und preisgünstig herstellbar hat sich ein Kulturgefäß erwiesen, bei dem die Mischelemente durch Flächenteile gebildet sind, die an die Dialyse-Membran und/oder an die Gasaustausch-Membran anschließend angeordnet sind. Eine besonders effektive und reproduzierbare Durchmischung der Zellkultur wird bei einer Ausbildung des Kulturgefäßes beobachtet, bei der die Membran und die Flächenteile einstückig ausgebildet sind und aus dem gleichen Material bestehen. Die Flächenteile können beispielsweise als von den Membranen abstehende Schaufeln ausgebildet sein. Insbesondere hat sich eine Ausführungsform der Gasaustausch-Membran als vorteilhaft erwiesen, bei der die Mischelemente als schaufelartig von der Membran abstehende Stege ausgebildet sind, wobei diese mit über ihre Länge verteilten und quer zur Längsrichtung verlaufenden Durchgangskanälen versehen sind. Bei einer Bewegung der Stege in einem flüssigen Medium, beispielsweise bei einer Rotation um eine auf der Längsachsenrichtung der Stege senkrecht stehenden Achse, erzeugen die Durchgangskanäle Luftblasen, die zu einer sehr guten Verwirbelung des zu durchmischenden Mediums beitragen. Besonders vorteilhaft ist eine Ausführungsform, bei der sich die Stege von einer Membran bis zu einer parallel dazu verlaufenden, gegenüberliegenden Membran erstrecken.

Als günstig hat sich auch ein Kulturgefäß erwiesen, bei dem die Gasaustausch-Membran verdickte Bereiche aufweist. Diese Bereiche können beispielsweise zur Probenentnahme aus der Zellkultur-Kammer bzw. zur Beimpfung der Zellkultur bzw. der Zellkulturen dienen. Dadurch, daß diese Bereiche verdickt ausgebildet sind, schließt sich die durch den Einstich der Probenentnahme- bzw. Impfnadel verursachte Öffnung nach dem Herausziehen der Nadel von selbst wieder. Es ist auch möglich, dieselbe Einstich-Öffnung mehrmals an derselben Stelle einzustechen.

Als besonders einfach herstellbar hat sich ein Kulturgefäß erwiesen, bei dem die Dialyse-Membran und die Gasaustausch-Membran das gleiche Material enthalten, insbesondere aus dem gleichen Material bestehen. Wesentliche Teile eines derartigen Kulturgefäßes lassen sich beispielsweise als einfache Gußoder Spritzgußteile herstellen. Unterschiedliche Eigenschaften, beispielsweise unterschiedliche Durchlässigkeiten für die Dialyse-Membran und die Gasaustausch-Membran können dadurch eingestellt werden, daß der einen Membran-Art Füllstoffe oder den beiden Membran-Arten unterschiedliche Füllstoffe zugesetzt werden. Die Füllstoffe können dabei beispielsweise mittels Tränken der entsprechenden Membran-Bereiche in geeignete Lösungen, die die Füllstoffe an sich in Form eines Vorproduktes enthalten, in die jeweiligen Membran-Bereiche eingebracht werden. Gegebenenfalls können die Füllstoffe nach dem Tränken in der Membran verfestigt werden.

Insbesondere im Hinblick auf eine hohe Zelldichte innerhalb der Zellkultur hat sich ein Kulturgefäß als vorteilhaft erwiesen, bei der die Membran gefaltet ausgebildet ist. Dadurch wird bei gegebener von der Membran überspannter Fläche, die Gesamtoberfläche der Membran vergrößert, so daß der Gasaustausch, insbesondere die Versorgung der Zellkultur mit Sauerstoff verbessert wird. Beispielsweise kann bei hohlzylindrischen Kulturgefäßen die Mantelfläche in Form eines Faltenbalgs ausgebildet sein. Derartige Faltenbälge sind einfach herzustellen.

Insbesondere im Hinblick auf die Formstablität des Kulturgefäßes und damit auf die Reproduzierbarkeit der damit erzielten Ergebnisse hat es sich auch als günstig erwiesen, die Gasaustausch-Membran und/oder die Dialyse-Membran mit mindestens einem, die Membran mechanisch stabilisierenden Stützelement zu versehen. Derartige Stützelemente, die beispielsweise bei den mechanischen Belastungen, wie sie bei einer Verwendung des Kulturgefäßes als Rollflasche -auf die Außenwandungen des Kulturgefäßes einwirken, die Form und die geometrische Anordnung der einzelnen Teile des Kulturgefäßes zueinander stabilisieren, werden insbesondere bei dünnwandigen und bei großen Flächen überspannenden Membranen bevorzugt.

Besonders bewährt hat sich ein Kulturgefäß, bei dem die Gasaustausch-Membran und/oder die Dialyse-Membran ein mechanisch stabiles Trägergerüst enthalten, das mit einem die Membran bildenden Material überspannt ist. Das Trägergerüst wird bevorzugt in Form eines Netzes oder eines Gitters ausgeführt, wobei als Trägerwerkstoff beispielsweise ein Metall oder ein mechanisch und chemisch stabilder Kunststoff gewählt werden kann; bevorzugt wird jedoch ein Trägergerüst, das aus dem gleichen Material besteht wie die Membran, von der es überspannt ist, wobei die mechanische Stabilität dadurch erreicht werden kann, daß das Trägergerüst dickwandiger als die Membran selbst ausgebildet ist. Dabei ragen die verdickten Bereiche des Trägergerüsts vorteilhafterweise von der Oberfläche der Membran in das Innere des Kulturgefäßes. Die dadurch entstehenden Flächenteile können bei der Bewegung des Kulturgefäßes als Mischelemente für die Zellkultur und/oder für das Nährmedium dienen. Die das Trägergerüst überspannende Membran kann beispielsweise durch Tränken des Trägergerüstes mit einem Material oder mit mehreren verschiedenen Materialien, insbesondere Kunststoffen oder geeignete Kunststoffe bildenden Vorprodukten, die zum Beispiel nach dem Tränken des Trägergerüstes ausgehärtet werden, hergestellt sein. Dabei können für die Gasaustausch-Membran und die Dialyse-Membran an die jeweiligen Erfordernisse angepaßte, unterschiedliche Membran-Materialien gewählt werden.

Es wird eine Ausführungsform des Kulturgefäßes bevorzugt, bei der für das Nährmedium eine Versorgungs-Kammer vorgesehen ist, die von der Zellkultur-Kammer durch eine Dialyse-Membran getrennt ist. Durch das Bereitstellen des Nährmediums in einer eigenen, der Zellkultur-Kammer benachbarten Versorgungs-Kammer wird eine Erneuerung oder Kontrolle des Nährmediums ermöglicht. Dadurch ist die Dialyse-Membran ständig mit Nährmedium in Kontakt und es wird eine stetige Versorgung der Zellkultur bzw. der Zellkulturen gewährleistet. Die Handhabung eines derartigen Kulturgefäßes, insbesondere Austausch und Auffüllen von Nährmedium oder die Entnahme von Proben, ist besonders einfach, wenn die Versorgungs-Kammer mit einer Einfüllöffnung versehen ist, durch die das flüssige Nährmedium in die Versorgungs-Kammer eingefüllt oder aus dieser entnommen werden kann.

Besonders einfach gestaltet sich ein Kulturgefäß, bei dem die Zellkultur-Kammer zwei sich in etwa gegenüberliegende Flächen aufweist, wobei die eine Fläche die Dialyse-Membran bildet. Beispielsweise können die beiden Flächen die Stirnseiten einer hohlzylindrischen Zellkultur-Kammer bilden. Die Fläche der einen Stirnseite kann dabei von der Dialyse-Membran, an die sich eine das Nährmedium enthaltende Versorgungseinheit anschließt, und die Fläche der anderen Stirnseite beispielsweise von der Gasaustausch-Membran gebildet sein, wobei bei einer Verwendung des Kulturgefäßes als Rollflasche die Mantelfläche als Abrollfläche dienen kann.

Besonders vorteilhaft bei einer derartigen Ausführungsform des Kulturgefäßes ist die Ausbildung der Membranen als einfache Folien oder Flachmembranen. Diese sind einfach herstellbar, leicht zu reinigen und zu sterilisieren. Weiterhin wird dadurch, daß die Membranen die Zellkulturkammer vertikal begrenzen, deren Handhabung, unabhängig von den übrigen Teilen des Kulturgefäßes, erleichtert und eine modulartige Ausbildung des Kulturgefäßes ermöglicht.

Es wird eine Ausführungsform des Kulturgefäßes bevorzugt, bei dem der Zellkultur-Kammer allseitig von der Dialyse-Membran oder der Gasaustausch-Membran begrenzt ist, wobei die Flächen der Dialyse-Membran und der Gasaustausch-Membran so groß gewählt sind, daß die Versorgung der Zellkultur mit Nährstoffen und mit den für die Zellatmung und für die Erhaltung der physiologischen Bedingungen notwendigen Gasen sowie der Abtransport der Stoffwechselprodukte gewährleistet ist. Bei einer Versorgung der Zellkultur bzw. der Zellkulturen mit Nährstoffen in ausreichender Menge und mit den für den Stoffwechsel erforderlichen Gasen sind hohe Zelldichten erzielbar, wenn gleichzeitig die dabei entstehenden Stoffwechselprodukte fortlaufend abtransportiert werden. Ein besonders schneller Gasaustausch und besonders hohe Zelldichten sind mit einem Kulturgefäß erzielbar, dessen gesamte Außenwand, abgesehen von eventuell erforderlichen Stützelementen, als Membran ausgebildet sind.

Bei Kulturgefäßen, die mindestens eine Versorgungs-Kammer aufweisen wird insbesondere im Hinblick auf eine einfache Handhabbarkeit eine Ausführungsform bevorzugt, die modulartig aus mehreren Einzelelementen zusammengesetzt ist, wobei die mindestens eine Versorgungs-Kammer und die mindestens eine Zellkultur-Kammer als Einzellelemente ausgebildet sind.

Ausführungsbeispiele der Erfindung sind in der Patentzeichnung dargestellt und werden nachstehend näher erläutert. In der Zeichnung zeigen in schematischer Darstellung:
- Figur 1: ein rollflaschenähnliches Kulturgefäß für Zellkulturen mit einer Zellkultur-Kammer und einer Versorgungs-Kammer im Längsschnitt,
- Figur 2: ein rollflaschenähnliches Kulturgefäß mit einer Zellkultur-Kammer und einer Versorgungseinheit im Längsschnitt,
- Figur 3: eine Draufsicht auf eine mit Mischelementen versehene Gasaustausch-Membran
- Figur 4: eine Seitenansicht auf eine mit Mischelementen versehene Gasaustausch-Membran
- Figur 5: einen Querschnitt durch ein rollflaschenähnliches Kulturgefäß mit einer Versorgungs-Kammer und einer darin angeordneten Zellkultur-Kammer,
- Figur 6: ein rollflaschenähnliches Kulturgefäß mit einer Zellkultur-Kammer und einer Versorgungs-Kammer, das im wesentlichen von Membranen begrenzt ist im Längschnitt und
- Figur 7: eine Konstruktionszeichnung eines erfindungsgemäßen, modulartig aufgebauten Kulturgefäßes mit einer gefalteten Gasversorgungs-Membran im Längsschnitt.

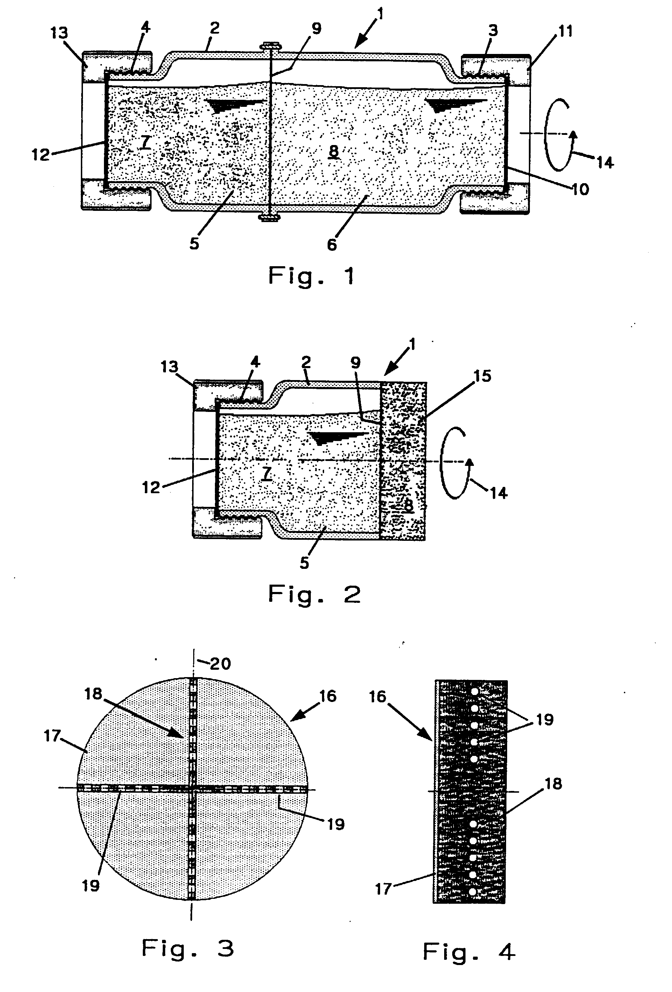

In den Figuren 1 bis 6 ist dem Kulturgefäß insgesamt die Bezugsziffer 1 zugeordnet. Das Kulturgefäß 1 ist rollflaschenähnlich, das heißt, im wesentlichen in Form eines Hohlzylinders ausgebildet. Dessen Mantelfläche wird von einem beidseitig offenen Glasrohr 2 gebildet, das im Bereich seiner Stirnseiten jeweils mit einem Außengewinde 3; 4 versehen ist. Das Kulturgefäß 1 weist zwei voneinander lösbare Kammern, nämlich eine Zellkultur-Kammer 5 und eine Versorgungs-Kammer 6 auf. Die Zellkultur-Kammer 5 enthält die zu kultivierende Zellkultur 7, während die Versorgungs-Kammer 6 ein Nährmedium 8 aufnimmt. Die beiden Kammern sind vertikal durch eine Dialyse-Membran 9, durch die Nährstoffe von der Versorgungs-Kammer 6 in die Zellkultur-Kammer 5 transportiert, und umgekehrt, Stoffwechselprodukte von der Zellkultur-Kammer 5 in die Versorgungs-Kammer 6 abtransportiert werden, voneinander getrennt. Die der Versorgungs-Kammer 6 zugeordnete-Stirnseite des Glasrohres 2 ist mit einer gasdurchlässigen, 0,3 mm dicken PTFE-Scheibe 10 verschlossen und wird mittels eines in das Außengewinde 3 des Glasrohres 2 eingreifenden ringförmigen Drehverschlusses 11 flüssigkeitsdicht gegen das Glasrohr 2 gepresst. Zum Nachfüllen oder Austauschen des Nährmediums 8 wird der Drehverschluß 11 geöffnet und die PTFE-Scheibe 10 abgenommen. Die der Zellkultur-Kammer 5 zugeordnete Stirnseite des Glasrohres 2 ist mit einer als Gasaustausch-Membran dienenden Silikon-Folie 12 verschlossen, die eine Dicke von ca. 0,5 mm aufweist. Die Silikon-Folie 12, die für Sauerstoff und für Kohlendioxidgas durchlässig ist, überspannt eine Fläche von ca. 10 cm². Sie wird mittels eines Schraubrings 13 flüssigkeits- und bakteriendicht mit dem Glasrohr 2 verbunden. Die Zellkultur-Kammer 5 vermag ein Volumen von ca. 60 ml aufzunehmen; das Gesamtvolumen der Versorgungs-Kammer 3 beträgt ca. 300 ml.

Das Kulturgefäß 1 ist, beispielsweise auf einer Rollen-Drehvorrichtung, um seine Längsachse rotierbar, wie dies durch den Richtungspfeil 14 angedeutet ist. Gleichzeitig mit der Rotation kann dem Kulturgefäß 1 eine langsame, zyklische Taumelbewegung aufgeprägt werden, bei der die Stirnseiten des Kulturgefäßes 1 relativ zueinander eine stetige Auf- und Abbewegung in Art einer Schaukelbewegung ausführen. Dabei findet eine Durchmischung des flüssigen Nährmediums 8 sowie des Zellkulturansatzes 7 statt, so daß durch die jeweiligen Membranen 9;12 eine gleichmäßige Versorgung der Zellkultur 7 mit den für die Zellatmung notwendigen Gasen und mit Nährstoffen aus dem Nährmedium 8 sowie ein stetiger Abtransport von Stoffwechselprodukten aus der Zellkultur 7 in das Nährmedium 8 bzw. in die Brutschrankatmosphäre sichergestellt wird.

Der für die Gasversorgung und -entsorgung der Zellkultur 7 notwendige Gasaustausch findet in erster Linie durch die Silikon-Folie 12 statt. Die Silikon-Folie 12 ist dabei so gewählt, daß sie weder durch Nährstoffe, noch durch Stoffwechselprodukte der Zellkultur 7 durchdrungen oder von diesen verstopft werden kann. Dadurch ist ein ungehinderter, freier Gasaustausch zwischen der Zellkultur-Kammer 5 und der sie umgebenden Brutschrankatmosphäre gewährleistet. Indirekt wird die Zellkultur auch über den durch die PTFE-Scheibe in die Versorgungs-Kammer eingetragenen Sauerstoff versorgt. Im konkreten Ausführungsbeispiel, bei der das Volumen des Zellkulturansatz 7 ca. 35 ml beträgt, werden im Ergebnis mehr als 10⁷ Zellen pro Milliliter Zellkulturansatz erwartet. Die Durchlässigkeit der Folie 12 für Sauerstoff ist auf ca. 3 mg/h eingestellt. Erfindungsgemäß gelangt beispielsweise der für die Vermehrung der Zellen erforderliche Sauerstoff von außen durch die Silikon-Folie 12 direkt in die Zellkultur 7 selbst, als auch in die über der Zellkultur 7 befindliche Atmosphäre, von wo er dann ebenfalls in die Zellkultur 7 eingetragen wird. Die Sauerstoffversorgung der Zellkultur 7 ist durch diese Art des Eintrags von Sauerstoff besonders effektiv und schnell und leicht von außen beeinflußbar. Auch die Konzentration des als Stoffwechsel produkt sich bildenden Kohlendioxidgases, das mit dem in der Zellkultur-Kammer 5 vorhandenen NaHCO₃ im Gleichgewicht steht, ist aufgrund des direkten Gasaustauschs zwischen der Zellkultur-Kammer 5 und der sie umgebenden Brutschrankatmosphäre über die Silikon-Folie 12 von außen schnell und relativ genau vorgebbar.

Sofern in den Figuren 2 bis 6 verwendete Bezugsziffern nicht näher erläutert sind, so betreffen sie gleiche oder äquivalente Bauteile oder Bestandteile des Kulturgefäßes 1, wie sie in Figur 1 anhand der gleichen Bezugsziffern beschrieben sind. Bei der in Figur 2 dargestellten Ausführungsform des Kulturgefäßes 1 ist eine Zellkultur-Kammer 5 vorgesehen, die durch eine Dialyse-Membran 9 von einer, ein gelartiges Nährmedium 8 enthaltenden, aus einer porösen Trägersubstanz bestehenden Versorgungseinheit 15 getrennt ist. Da in diesem Fall die Dialyse-Membran 9 an der im wesentlichen festen Versorgungseinheit 15 anliegt und dabei durch diese stabilisiert wird, kann sie sehr dünn, nämlich 0,01 mm, ausgelegt sein. Dadurch findet der Austausch der Nährstoffe bzw. der Soffwechselprodukte sehr rasch und ungehindert statt. Die für die Zellatmung notwendigen Gase werden der Zellkultur 7 wiederum durch eine ca. 0,5 mm dicke Silikon-Folie 12 zugeführt; umgekehrt wird gleichzeitig die beim Verbrauch des Sauerstoffs durch die Zellkultur 7 entstehende CO₂-Gasmenge über die Silikon-Folie 9 abgeführt.

Die in Figur 3 dargestellte Gasaustausch-Membran 16 besteht aus einer ca. 0,2 mm dicken Silikon-Folie 17, die mit Mischelementen versehen ist, die in Form sich kreuzender, etwa 2 mm hoher Silikon-Stege 18 ausgebildet sind. Die Silikon-Folie 17 wird derart in das Kulturgefäß 1 eingesetzt, daß die Silikon-Stege 18 nach innen in das zu durchmischende Medium (Zellkultur und/oder Nährmedium) ragen. Die Silikon-Stege 18 sind mit quer zur Längsrichtung verlaufenden Durchgangskanälen 19 versehen, die über die Länge der Stege 18 etwa gleichmäßig verteilt sind. Diese Durchgangskanäle 19 tragen zu einer besseren Durchmischung des Mediums bei. Aus der in Figur 4 dargestellten Seitenansicht in einem Schnitt durch die Membran 17 entlang der Linie 20 (Figur 3) ist ersichtlich, daß die Stege 18 über ihre Länge gleichmäßig verteilte, kreisrunde und quer zur Achsrichtung verlaufende Kanäle 19 mit einem Durchmesser von ca. 1 mm aufweisen. Vorteilhafterweise ist die Höhe der Stege 18 so gewählt, daß diese von der Membran 17 bis zur gegenüberliegenden Dialysemembran (in der Figur nicht dargestellt) ragen und so die (ebenfalls nicht dargestellte) Zellkulturkammer in mehrere Einzelkammern unterteilt wird, in denen die Zellkulturen vor allem durch die Luftblasen durchmischt werden, die beim Rotieren der Membran 17 aufgrund der Kanäle 19 erzeugt werden.

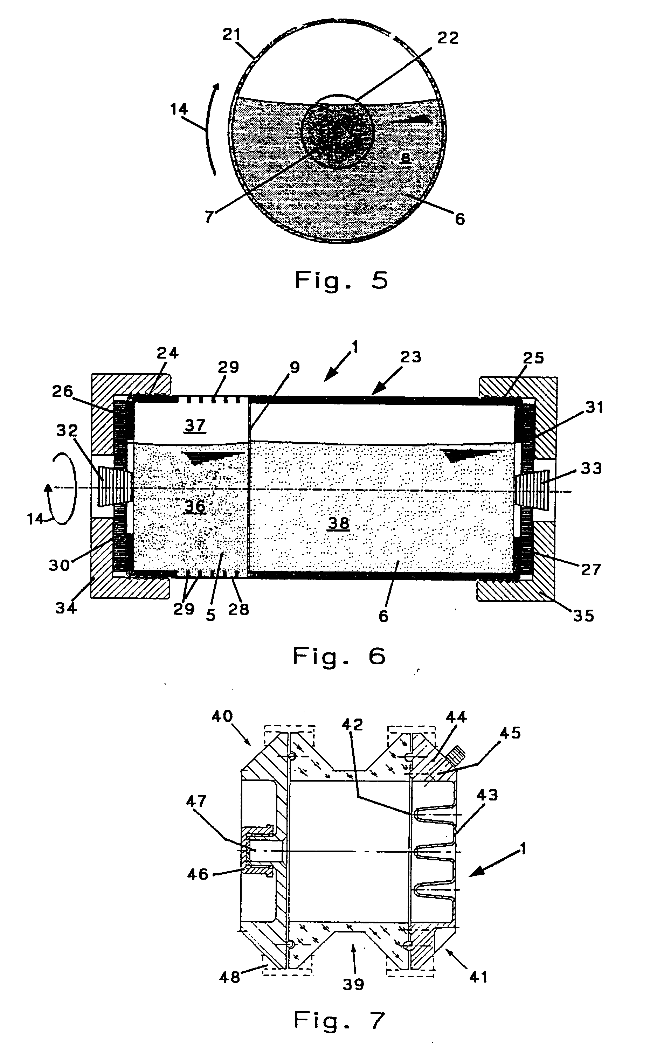

Das in Figur 5 dargestellte Kulturgefäß 1 besteht im wesentlichen aus einem Silikonrohr 21, das, die Versorgungs-Kammer 6 bildend, bei waagerechter Orientierung bis etwas über seine halbe Höhe mit Nährmedium 8 gefüllt ist. Innerhalb des Silikonrohres 21 und koaxial zu diesem verläuft ein mit dem Zellkulturansatz 7 teilweise gefüllter Dialyseschlauch 22, der sich von einer Stirnseite des Silikonrohres 21 zu der gegenüberliegenden Stirnseite erstreckt, wobei die Stirnseiten sowohl des Silikonrohres 21 als auch die des Dialyseschlauchs 22 bündig miteinander abschließen und jeweils durch eine gemeinsame Silikon-Folie (in der Figur nicht dargestellt) nach außen verschlossen sind. Sowohl über das Silikonrohr 21, als auch Über die Silikon-Folie erfolgt sowohl ein Gasaustausch zwischen der das Kulturgefäß 1 umgebenden Brutschrankatmosphäre und dem Innenraum der Versorgungs-Kammer 6, als auch direkt mit der Zellkultur 7. Mit den nicht gasförmigen Nährstoffen wird die Zellkultur 7 über die als semipermeable Membran ausgebildete Wandung des Dialyseschlauches 22 versorgt.

Bei der in Figur 6 dargestellten Ausführungsform eines erfindungsgemäßen Kulturgefäßes 1 ist sowohl die Zellkultur-Kammer 5 als auch die Versorgungs-Kammer 6 innerhalb eines Silikonrohres 23 angeordnet. Sie sind voneinander durch eine flache, semipermeable Dialysemembran 9 getrennt. Das Silikonrohr 23 ist im Bereich der Stirnseiten mit Außengewinden 24;25 und mit innen gerichteten Flanschen 26;27 versehen ist. Die Wandstärke des Silikonrohres 23 beträgt ca. 3 mm. Der die Zellkultur-Kammer 5 umgebende Teil des Silikonrohres 23 weist gleichmäßig verteilte Durchbrüche auf, die etwa zwei Drittel der Manteloberfläche dieses Teils des Silikonrohres 23 einnehmen und die von außen mit einer 0,38 mm dicken Silikonfolie 28 überspannt sind. Die in diesem Teil der Mantelfläche des Silikonrohres 23 verbleibenden, miteinander verbundenen Stege 29 ragen von der Silikonfolie 28 aus in das Innere der Zellkultur-Kammer 5 hinein und bilden ein zusammenhängendes Gitter. Sie verleihen dadurch der Zellkultur-Kammer 5 eine für die bei der Zellkultivierung auftretenden Belastungen ausreichende mechanische Stabilität. Das Kulturgefäß 1 hat eine Länge von ca. 15 cm, einen Außendurchmesser von ca. 5 cm und vermag insgesamt ein Volumen von ca. 300 ml, wovon ca. 60 ml auf die Zellkultur-Kammer 5 entfallen, aufzunehmen. Die Mantelfläche des Silikonrohres 23 insgesamt beträgt etwa 240 cm². Die Stirnseiten des Silikonrohres 23 sind jeweils mit einer eine Mittenbohrung aufweisenden Ringscheibe 30;31 aus stabilem Kunststoff verschlossen. Die Ringscheiben 30;31, deren Mittenbohrungen jeweils mit einem Gummistöpsel 32;33 verschließbar sind, werden mittels in die Außengewinde 25;26 des Kulturgefäßes 1 eingreifenden, ringförmigen Schraubdeckeln 34;35 flüssigkeitsdicht gegen die Flansche 26;27 des Silikonrohres 23 gepreßt. Die Gummistöpsel 32;33 sind über die Öffnungen der Schraubdeckel 34;35 zugänglich. Das Kulturgefäß 1 ist um seine Längsachse rotierbar, wie dies mit dem Richtungspfeil 14 angedeutet ist.

In die Kultur-Kammer 5 werden ca 35 ml Zellkulturansatz 36 eingefüllt und die Zellkultur-Kammer 5 anschließend mittels des Gummistöpsels 32 verschlossen. Dabei wird eine Luftblase 37 mit einem Volumen von ca. 25 ml in dem Zellkultur-Kammer 5 eingeschlossen. Gleichzeitig wird die Versorgungs-Kammer 6 bis etwa zu ihrer halben Höhe (bei waagerechter Orientierung) mit Nährmedium 38 für den Zellkulturansatz 36 gefüllt. Die Zellkultivierung wird in einem NaHCO₃-Puffer-abhängigen Medium vorgenommen. Zur Aufrechterhaltung des Puffersystems erfolgt die Kultivierung in einem in der Figur nicht dargestellten Brutschrank mit vorgegebener CO₂- und O₂-Atmosphäre, hoher Luftfeuchtigkeit und definierter Temperatur. Während der Rotation des Kulturgefäßes 1 wird die Dialysemembran 9 von dem Nährmedium 38 umspült. Dabei werden Nährstoffe von der Versorgungs-Kammer 2 in die Zellkultur (bzw. in den Zellkulturansatz; beides Bezugsziffer 36) transportiert und gleichzeitig von dort Stoffwechselprodukte in die Versorgungs-Kammer 2 abtransportiert. Der in der Brutschrank-Atmosphäre enthaltene Sauerstoff gelangt durch die dünne Silikon-folie 28, deren Durchlässigkeit für Sauerstoff ausreichend ist, um den Sauerstoffbedarf der Zellkultur 36 von mindestens 0,01 mg/h pro 10⁷ Zellen zu decken, direkt sowohl in die Zellkultur 36, als auch in die darüber befindliche Luftblase 37. Daneben werden auch geringere Mengen an Sauerstoff von der Versorgungs-Kammer 6 aus durch die Dialysemembran 9 in die Zellkultur 36 eingetragen. Das beim Verbrauch des Sauerstoffs entstehende Kohlendioxidgas wird ebenfalls überwiegend durch die Silikonfolie 28 aus dem Kulturgefäß 1 entfernt. Die Durchlässsigkeit der Silikonfolie 28 für Kohlendioxidgas ist wesentlich höher als diejenige für Sauerstoff, so daß innerhalb der Versorgungs-Kammer 5 insofern kein Überdruck entstehen kann. Für Flüssigkeiten und für die Zellkultur 36 eventuell kontaminierende Keime, wie Bakterien, Pilze oder Sporen ist die Silikonfolie 28 dagegen undurchlässig. Während der Kultivierung können über die jeweiligen Gummistöpsel 32;33 Proben entnommen, die Zellkultur 36 beimpft oder das Nährmedium 38 geprüft oder ausgetauscht werden.

Zum Zweck einer guten Durchmischung sowohl der Zellkultur 36 als auch des Nährmediums 38 wird das Kulturgefäß 1 mit einer Umdrehungsgeschwindigkeit von ca. 34 U/min um seine Längsachse rotiert, wobei die Rotation 14 von einer langsamen, zyklischen Taumelbewegung des Kulturgefäßes 1 überlagert wird, bei der die Stirnseiten des Silikonrohres 23 relativ zueinander eine stetige Auf- und Abbewegung in Art einer Schaukelbewegung ausführen. Dabei bewegt sich infolge ihres Auftriebs in der Zellkultur 36 auch die Luftblase 37 innerhalb der Zellkultur-Kammer 5 auf und ab und unterstützt damit die Durchmischung der Zellkultur 36 und damit deren gleichmäßige Versorung mit Nährstoffen, insbesondere mit Sauerstoff, besonders wirkungsvoll.

In Figur 7 ist ein erfindungsgemäßes Kulturgefäß 1 in einem Maßstab von 1:1 dargestellt. Es ist modulartig aus einem hülsenförmigen, aus Polysulfon bestehenden und das Nährmedium aufnehmenden Mittelteil 39, einer die eine Stirnseite des Mittelteils 39 flüssigkeitsdicht verschließenden Abschlußplatte 40 und einem, an der gegenüberliegenden Stirnseite des Mittelteils 39 angeordneten und den Zellkulturansatz aufnehmenden Zellkultur-Kammer 41 zusammengesetzt. Der das Nährmedium für die Zellkultur aufnehmende Mittelteil 39 ist von der Zellkultur-Kammer 41 durch eine semipermeable Dialysemembran 42 getrennt. Die Dialysemembran 42 ist der Zellkultur-Kammer 41 zugeordnet, die im übrigen aus Silikon besteht. Die der Dialysemembran 42 gegenüberliegende Seite der Zellkultur-Kammer 41 ist als in Art eines Faltenbalgs gefaltetes, dünnwandiges Silikonformteil 43 ausgebildet, während die Seitenwand aus einem dickwandigen, die Zellkultur-Kammer 41 mechanisch stabilisierenden Silikonring 44 besteht. Durch die Wandung des Silikonrings 44 erstreckt sich eine verschließbare Einfüllöffnung 45 in die Zellkultur-Kammer 41. Das Volumen der Zellkultur-Kammer 41 beträgt ca. 35 ml. Das Silikonformteil 43 weist eine Wandstärke von 0,2 mm auf und ist für Sauerstoff derart durchlässig, daß auch bei höherer Zelldichte als 10⁷ Zellen/ml keine Sauerstoffmangelversorgung auftritt. Um seine Oberfläche zu vergrößern und damit die Menge des hindurchdiffundierenden Sauerstoffs weiter zu erhöhen ist das Silikonformteil 43 in Form eines Faltenbalgs ausgebildet. Zum Einfüllen des Nährmediums weist die Abschlußplatte 40 einen mit einer Kappe 46 verschließbaren Einfüllstutzen 47 auf. Die einzelnen Module 39;40;41 des Kulturgefäßes 1 werden mittels an ihrem Umfang angreifender Klammern 48 zusammengehalten.

Das erfindungsgemäße Kulturgefäß 1 ist besonders einfach handhabbar; mit ihm sind hochreine Zellkulturen mit Zelldichten von mehr als 10⁷ Zellen/ml, und, im Falle von Hybridomzellen, Konzentrationen von monoklonalen Antikörpern erreichbar, die mindestens 10fach höher als die mit normalen Standkulturen erzielbaren Konzentrationen liegen.

## Patentansprüche

1. Kulturgefäß (1) für Zellkulturen mit mindestens einer die Zellkultur (7) aufnehmenden Zellkultur-Kammer (5; 41), die durch eine Dialyse-Membran (9; 22; 42) von einem einzubringenden Nährmedium (8; 38) abgetrennt ist, wobei durch die Dialyse-Membran (9; 22; 42) Nährstoffe in die Zellkultur-Kammer (5; 41) transportiert und von der Zellkultur-Kammer (5; 41) Stoffwechselprodukte in das Nährmedium abtransportiert werden, und mit einer Zuund Abführung für die bei der Zellkultivierung benötigten und entstehenden Gase, dadurch gekennzeichnet, daß als Gas-Zu- und Abführung eine für die Flüssigkeiten und für die Zellkulturen kontaminierende Keime undurchlässige Gasaustausch-Membran (12; 17; 28; 43) vorgesehen ist, die die Zellkultur-Kammer (5; 41) teilweise begrenzt und daß in der Zellkultur-Kammer (5; 41) Mischelemente (18) eingesetzt sind.

2. Kulturgefäß nach Anspruch 1, dadurch gekennzeichnet, daß die Gasaustausch-Membran (12; 17; 28; 43) aus Silikon oder aus einem mikroporösen, hydrophoben oder hydrophobierten Material besteht und eine Dicke zwischen 0,1 mm und 1 mm aufweist.

3. Kulturgefäß nach Anspruch 1 oder 2, dadurch gekennzeichnet, daß die Gasaustausch-Membran (12; 17; 28; 43) eine Fläche von mindestens 5 cm² aufweist.

4. Kulturgefäß nach einem der Ansprüche 1 bis 3, dadurch gekennzeichnet, daß die Mischelemente durch Flächenteile (18) gebildet sind, die an die Dialyse-Membran und/oder an die Gasaustausch-Membran (12; 17; 28; 43) anschließend angeordnet sind.

5. Kulturgefäß nach Anspruch 4, dadurch gekennzeichnet, daß die Flächenteile (18) aus demselben Material bestehen wie die Membran (12; 17; 28; 43; 9; 22; 42).

6. Kulturgefäß nach Anspruch 4 oder 5, dadurch gekennzeichnet, daß die Flächenteile (18) und die Membran (12; 17; 28; 43; 9; 22; 42) einstückig ausgebildet sind.

7. Kulturgefäß nach einem oder mehreren der Ansprüche 1 bis 6, dadurch gekennzeichnet, daß an der Gasaustausch-Membran (12; 17; 28; 43) verdickte Bereiche vorgesehen sind.

8. Kulturgefäß nach einem oder mehreren der Ansprüche 1 bis 7, dadurch gekennzeichnet, daß die Dialyse-Membran (9; 22; 42) und die Gasaustausch-Membran (12; 17; 28; 43) das gleiche Material enthalten.

9. Kulturgefäß nach Anspruch 8, dadurch gekennzeichnet, daß die Dialyse-Membran (9; 22; 42) und/oder die Gasaustausch-Membran (12; 17; 28; 43) Füllstoffe enthalten.

10. Kulturgefäß nach einem oder mehreren der Ansprüche 1 bis 9, dadurch gekennzeichnet, daß die Membran (12; 17; 28; 43; 9; 22; 42), insbesondere die Gasaustausch-Membran (12; 17; 28; 43), gefaltet ausgebildet ist.

11. Kulturgefäß nach einem oder mehreren der Ansprüche 1 bis 10, dadurch gekennzeichnet, daß die die Gasaustausch-Membran (12; 17; 28; 43) und/oder die Dialyse-Membran (9; 22; 42) mit mindestens einem die Membran mechanisch stabilisierenden Stützelement (15; 23; 29) versehen ist.

12. Kulturgefäß nach einem oder mehreren der Ansprüche 1 bis 11, dadurch gekennzeichnet, daß die Gasaustausch-Membran (12; 17; 28; 43) und/oder die Dialyse-Membran (9; 22; 42) ein Trägergerüst (29) enthalten, das mit einem die Membran bildenden Material (28) überspannt ist.

13. Kulturgefäß nach einem oder mehreren der Ansprüche 1 bis 12, dadurch gekennzeichnet, daß für das Nährmedium (8; 38) mindestens eine Versorgungskammer (6; 39) vorgesehen ist, die von der Zellkultur-Kammer (5) durch eine Dialyse-Membran (9; 22; 42) getrennt ist.

14. Kulturgefäß nach Anspruch 13, dadurch gekennzeichnet, daß in der Außenwand der Versorgungskammer (6; 39) eine Einfüllöffnung (45) für das Nährmedium (8; 38) vorgesehen ist.

15. Kulturgefäß nach einem oder mehreren der Ansprüche 1 bis 14 dadurch gekennzeichnet, daß die Zellkultur-Kammer (5) zwei in etwa gegenüberliegende Begrenzungsflächen (9; 12; 42; 43) aufweist, wovon die eine von der Dialyse-Membran (9; 22; 42) gebildet wird.

16. Kulturgefäß nach einem oder mehreren der Ansprüche 1 bis 15, dadurch gekennzeichnet, daß das Kulturgefäß (1) allseitig von Membranen (12; 17; 28; 43; 9; 21; 22; 42) begrenzt ist, von denen ein Teil aus der Dialyse-Membran (9; 22; 42) und ein Teil aus der Gasaustausch-Membran (12; 17; 28; 43) besteht, wobei die Flächen der Dialyse-Membran (9; 22; 42) und der Gasaustausch-Membran (12; 17; 28; 43) so groß gewählt sind, daß die Versorgung der Zellkultur (7; 36) mit Nährstoffen und mit den für die Zellatmung und für die Erhaltung der physiologischen Bedingungen notwendigen Gase sowie der Abtransport der Stoffwechselprodukte gewährleistet ist.

17. Kulturgefäß nach Anspruch 13 und einem oder mehreren der Ansprüche 1 bis 12 und 14 bis 16, dadurch gekennzeichnet, daß es modulartig aus mehreren Einzelelementen (39; 40; 41) zusammengesetzt ist, wobei die mindestens eine Versorgungskammer (39) und die mindestens eine Zellkultur-Kammer (41) als Einzelelemente ausgebildet sind.

## Claims

1. A culture vessel (1) for cell cultures with at least one cell culture chamber (5; 41) receiving the cell culture (7), which chamber (5; 41) is separated by a dialysis membrane (9; 22; 42) from a nutrient medium (8; 38) which is to be introduced, wherein nutrients are transported into the cell culture chamber (5; 41) through the dialysis membrane (9; 22; 42) and metabolic products are transported away in the nutrient medium from the cell culture chamber (5; 41), and with a supply- and removal arrangement for the gases required and arising in the cell cultivation, characterised in that as gas supply and removal arrangement, a gas exchange membrane (12; 17; 28; 43) is provided which is impermeable to the fluids and to germs contaminating the cell cultures, which gas exchange membrane (12; 17; 28; 43) partially delimits the cell culture chamber (5; 41) and that mixing members (18) are inserted in the cell culture chamber (5; 41).

2. A culture vessel according to Claim 1, characterised in that the gas exchange membrane (12; 17; 28; 43) consists of silicon or of a microporous, hydrophobic material or material rendered hydrophobic and has a thickness between 0.1 mm and 1 mm.

3. A culture vessel according to Claim 1 or 2, characterised in that the gas exchange membrane (12; 17; 28; 43) has an area of at least 5 cm².

4. A culture vessel according to one of Claims 1 to 3, characterised in that the mixing members are formed by area pieces (18) which are subsequently arranged against the dialysis membrane and/or against the gas exchange membrane (12; 17; 28; 43).

5. A culture vessel according to Claim 4, characterised in that the area pieces (18) consist of the same material as the membrane (12; 17; 28; 43; 9; 22; 42).

6. A culture vessel according to Claim 4 or 5, characterised in that the area pieces (18) and the membrane (12; 17; 28; 43; 9; 22; 42) are constructed in one piece.

7. A culture vessel according to one or more of Claims 1 to 6, characterised in that thickened regions are provided on the gas exchange membrane (12; 17; 28; 43).

8. A culture vessel according to one or more of Claims 1 to 7, characterised in that the dialysis membrane (9; 22; 42) and the gas exchange membrane (12; 17; 28; 43) contain the same material.

9. A culture vessel according to Claim 8, characterised in that the dialysis membrane (9; 22; 42) and/or the gas exchange membrane (12; 17; 28; 43) contain fillers.

10. A culture vessel according to one or more of Claims 1 to 9, characterised in that the membrane (12; 17; 28; 43; 9; 22; 42), in particular the gas exchange membrane (12; 17; 28; 43) is constructed in folded form.

11. A culture vessel according to one or more of Claims 1 to 10, characterised in that the gas exchange membrane (12; 17; 28; 43) and/or the dialysis membrane (9; 22; 42) is provided with at least one support member (15; 23; 29) stabilising the membrane mechanically.

12. A culture vessel according to one or more of Claims 1 to 11, characterised in that the gas exchange membrane (12; 17; 28; 43) and/or the dialysis membrane (9; 22; 42) contain a support structure (29) which is stretched over by a material (28) forming the membrane.

13. A culture vessel according to one or more of Claims 1 to 12, characterised in that at least one supply chamber (6; 39) is provided for the nutrient medium (8; 38), which chamber (6; 39) is separated from the cell culture chamber (5) by a dialysis membrane (9; 22; 42).

14. A culture vessel according to Claim 13, characterised in that a filling opening (45) for the nutrient medium (8; 38) is provided in the outer wall of the supply chamber (6; 39).

15. A culture vessel according to one or more of Claims 1 to 14, characterised in that the cell culture chamber (5) has two areas of contact (9; 12; 42; 43) lying approximately opposite each other, one of which is formed by the dialysis membrane (9; 22; 42).

16. A culture vessel according to one or more of Claims 1 to 15, characterised in that the culture vessel (1) is delimited on all sides by membranes (12; 17; 28; 43; 9; 21; 22; 42), one part of which consists of the dialysis membrane (9; 22; 42) and one part of which consists of the gas exchange membrane (12; 17; 28; 43), wherein the areas of the dialysis membrane (9; 22; 42) and of the gas exchange membrane (12; 17; 28; 43) are selected so be so great that the supply of the cell culture (7; 36) with nutrients and with the gases necessary for the cell respiration and for maintaining the physiological conditions and also the transporting away of the metabolic products is ensured.

17. A culture vessel according to Claim 13 and one or more of Claims 1 to 12 and 14 to 16, characterised in that it is composed in a modular manner from several individual members (39; 40; 41), wherein the at least one supply chamber (39) and the at least one cell culture chamber (41) are constructed as individual members.

## Revendications

1. Bouteille de culture (1) pour des cultures cellulaires, comportant au moins une chambre de culture (5 ; 41) recevant la culture cellulaire (7) et séparée par une membrane de dialyse (9 ; 22 ; 42) vis-à-vis d'un milieu de culture à introduire (8 ; 38), dans laquelle des substances nutritives sont transportées à travers la membrane de dialyse (9 ; 22 ; 42) jusque dans la chambre de culture (5 ; 41), et des produits du métabolisme sont évacués à travers ladite membrane de dialyse depuis la chambre de culture (5 ; 41) jusque dans le milieu de culture, et comportant une amenée/évacuation pour les gaz qui sont nécessaires et ceux qui se développent pendant la culture des cellules, caractérisée en ce qu'il est prévu en tant qu'amenée/évacuation de gaz une membrane d'échange de gaz (12 ; 17 ; 28 ; 43) imperméable aux liquides et aux germes contaminant les cultures cellulaires, ladite membrane délimitant partiellement la chambre de culture (5 ; 41), et en ce que des éléments mélangeurs (18) sont mis en place dans la chambre de culture (5 ; 41).

2. Bouteille de culture selon la revendication 1, caractérisée en ce que la membrane d'échange de gaz (12 ; 17 ; 28 ; 43) est constituée en silicone ou en un matériau microporeux, hydrophobe ou rendu hydrophobe, et présente une épaisseur entre 0,1 mm et 1 mm.

3. Bouteille de culture selon l'une ou l'autre des revendications 1 et 2, caractérisée en ce que la membrane d'échange de gaz (12 ; 17 ; 28 ; 43) présente une surface d'au moins 5 cm².

4. Bouteille de culture selon l'une quelconque des revendications 1 à 3, caractérisée en ce que les éléments mélangeurs sont formés par des parties de surface (18) qui sont agencées de manière à se raccorder à la membrane de dialyse et/ou à la membrane d'échange de gaz (12 ; 17 ; 28 ; 43).

5. Bouteille de culture selon la revendication 4, caractérisée en ce que les parties de surface (18) sont constituées du même matériau que la membrane (12 ; 17 ; 28 ; 43 ; 9 ; 22 ; 42).

6. Bouteille de culture selon l'une ou l'autre des revendications 4 et 5, caractérisée en ce que les parties de surface (18) et la membrane (12 ; 17 ; 28 ; 43 ; 9 ; 22 ; 42) sont réalisées en une seule pièce.

7. Bouteille de culture selon l'une ou plusieurs des revendications 1 à6, caractérisée en ce qu'il est prévu des régions épaissies sur la membrane d'échange de gaz (12 ; 17 ; 28 ; 43).

8. Bouteille de culture selon l'une ou plusieurs des revendications 1 à 7, caractérisée en ce que la membrane de dialyse (9 ; 22 ; 42) et la membrane d'échange de gaz (12 ; 17 ; 28 ; 43) contiennent le même matériau.

9. Bouteille de culture selon la revendication 8, caractérisée en ce que la membrane de dialyse (9 ; 22 ; 42) et/ou la membrane d'échange de gaz (12 ; 17 ; 28 ; 43) contiennent des matières de charge.

10. Bouteille de culture selon l'une ou plusieurs des revendications 1 à 9, caractérisée en ce que la membrane (12 ; 17 ; 28 ; 43 ; 9 ; 22 ; 42), en particulier la membrane d'échange de gaz (12 ; 17 ; 28 ; 43), est réalisée de façon plissée.

11. Bouteille de culture selon l'une ou plusieurs des revendications 1 à 10, caractérisée en ce que la membrane d'échange de gaz (12 ; 17 ; 28 ; 43) et/ou la membrane de dialyse (9 ; 22 ; 42) est pourvue d'au moins un élément de soutien (15 ; 23 ; 29) qui stabilise mécaniquement la membrane.

12. Bouteille de culture selon l'une ou plusieurs des revendications 1 à 11, caractérisée en ce que la membrane d'échange de gaz (12 ; 17 ; 28 ; 43) et/ou la membrane de dialyse (9 ; 22 ; 42) comportent une structure de support (29) qui est recouverte d'un matériau (28) formant la membrane.

13. Bouteille de culture selon l'une ou plusieurs des revendications 1 à 12, caractérisée en ce qu'il est prévu au moins une chambre d'alimentation (6 ; 39) pour le milieu de culture (8 ; 38), qui est séparée de la chambre de culture (5) par une membrane de dialyse (9 ; 22 ; 42).

14. Bouteille de culture selon la revendication 13, caractérisée en ce qu'il est prévu une ouverture de remplissage (45) pour le milieu de culture (8 ; 38) dans la paroi extérieure de la chambre d'alimentation (6 ; 39).

15. Bouteille de culture selon l'une ou plusieurs des revendications 1 à 14, caractérisée en ce que la chambre de culture (5) présente deux surfaces de délimitation (9 ; 12 ; 42 ; 43) approximativement en vis-à-vis, dont l'une est formée par la membrane de dialyse (9 ; 22 ; 42).

16. Bouteille de culture selon l'une ou plusieurs des revendications 1 à 15, caractérisée en ce que la bouteille de culture (1) est délimitée de tous les côtés par des membranes (12 ; 17 ; 28 ; 43 ; 9 ; 21 ; 22 ; 42), dont une partie est constituée par la membrane de dialyse (9 ; 22 ; 42) et une partie par la membrane d'échange de gaz (12 ; 17 ; 28 ; 43), les surfaces de la membrane de dialyse (9 ; 22 ; 42) et de la membrane d'échange de gaz (12 ; 17 ; 28 ; 43) étant choisies d'une taille si importante que l'alimentation de la culture cellulaire (7 ; 36) en substances nutritives et en gaz nécessaires pour la respiration cellulaire et pour le maintien des conditions physiologiques, ainsi que l'évacuation des produits du métabolisme sont assurées.

17. Bouteille de culture selon la revendication 13 ou selon l'une ou plusieurs des revendications 1 à 12 et 14 à 16, caractérisée en ce qu'elle est composée de façon modulaire de plusieurs éléments individuels (39 ; 40 ; 41), ladite au moins une chambre d'alimentation (39) et ladite au moins une chambre de culture (41) étant réalisées en tant qu'éléments individuels.
